# EUROPÄISCHE PATENTSCHRIFT

(11) **EP 0 538 641 B1**
(45) Veröffentlichungstag und Bekanntmachung des Hinweises auf die Patenterteilung: **09.07.1997**
(21) Anmeldenummer: 92116377.0
(22) Anmeldetag: 24.09.1992
(51) Int. Cl.: A61B 17/36

(54) **Chirurgische Laservorrichtung mit einem Rauchabzugssystem**
Surgical laser device with a smoke evacuation system
Laser chirurgical avec un système d'évacuation des fumées

(30) Priorität: 24.09.1991 US 764842
(43) Veröffentlichungstag der Anmeldung: 28.04.1993
(73) Patentinhaber: COSMESCU, Ioan, Phoenix, AZ 85022 (US)
(72) Erfinder: COSMESCU, Ioan, Phoenix, AZ 85022 (US)
(74) Vertreter: Hieke, Kurt

(56) Entgegenhaltungen:
- DE-A- 3 209 444
- US-A- 4 735 603
- US-A- 4 850 352
- US-A- 4 998 527
- US-A- 5 108 389

## Beschreibung

Die Erfindung bezieht sich auf eine chirurgische Laservorrichtung gemäß dem einleitenden Teil des Patentanspruchs 1.

Chirurgische Laservorrichtungen dieser Art sind aus der DE-A-32 09 444 bekannt.

Diese bekannten chirurgischen Laservorrichtungen sind insofern vorteilhaft, als sie nur einen einzigen Schnitt am Patienten für die Anbringung der Laservorrichtung einschließlich des Rauchabzugssystems erfordern. Nachteilig ist bei ihnen aber, daß der Gasabzug aus dem Bereich der chirurgischen Laservorrichtung manuell vom Operateur gesteuert wird, für den die Durchführung der Operation dadurch erheblich erschwert ist.

Aus dem U.S.-Patent Nr. 4,735,603 ist ein System und ein Verfahren zum Rauchabzug bei einer Laseranwendung bekannt. Bei diesem System wird an einem Laparoskop eine Einblasvorrichtung für CO₂ angebracht, um eine ausreichende Auswölbung der Körperhöhle des Patienten wahrend des chirurgischen Eingriffs aufrechtzuerhalten, sowie eine getrennte Vakuumquelle für den Abzug des Rauchs aus der Körperhöhle. Dieses System erfordert wenigstens einen zusätzlichen Schnitt am Patienten für die Vakuumquelle. Die Vakuumquelle entfernt CO₂ aus der Körperhöhle, das mit Rauch, Wasserdampf und Teilchen kontaminiert ist. Dieses kontaminierte CO₂ wird gefiltert und der CO₂-Einblasvorrichtung wieder zugeführt, die das so behandelte CO₂ wieder in die Körperhöhle des Patienten zurückpumpt.

Die Vakuumquelle ist ein separates Instrument und erfordert einen zusätzlichen Schnitt am Patienten, damit die Vakuumquelle in der Nähe des durchzuführenden chirurgischen Eiungriffs in die Körperhöhle eingeführt werden kann. Eine zusätzliche medizinische Fachkraft wird benötigt, um die Vakuumquelle in geeigneter Weise zu positionieren und während des chirurgischen Eingriffs die richtige Positionierung beizubehalten, wodurch der chirurgische Eingriff mit beachtlichen zusätzlichen Kosten belastet wird. Diese Art von Rauchahzugssystem wird generell von Hand eingeschaltet und kontinuierlich während des gesamten laserchirurgischen Eingriffs betrieben. Dieser kontinuierliche Betrieb des Rauchabzugssystems während des chirurgischen Eingriffs erzeugt einen konstanten Lärm und verbraucht ständig elektrische Energie. Zusätzlich hat der kontinuierlich auf das Filterelement des Rauchabzugssystems einwirkende Luftdruck die Folge, daß das Filterelement des Systems gewöhnlich gesättigt oder überladen wird, wodurch die toxischen Gase, von welchen angenommen wird, sie würden aus dem Bereich des chirurgischen Eingriffs entfernt, unkontrolliert in den Operationsraum entweichen, statt nach außen abgeleitet zu werden, wie man es eigentlich vom Rauchabzugssystem erwartet.

Der Erfindung liegt die Aufgabe zugrunde, die nur einen einzigen Schnitt am Patienten erfordernden chirurgischen Laservorrichtungen gemäß dem einleitenden Teil des Patentanspruchs 1 so zu gestalten, daß das Rauchabzugssystem automatisch bei der Aktivierung des Laserschneidstrahls der chirurgischen Laservorrichtung aktiviert wird.

Diese Aufgabe wird durch die Merkmale des Patentanspruchs 1 gelöst.

Die Unteransprüche 2 bis 23 haben bevorzugte Ausführungsformen der chirurgischen Laservorrichtung nach Anspruch 1 zum Gegenstand.

Zur Erläuterung wird nachfolgend als laserchirurgisches Instrument ein Laparoskop beschrieben. Dieses Laparoskop ist mit einem externen Fitting versehen, das normalerweise mit einer CO₂-Einblasvorrichtung verbunden werden kann. Das CO₂ wird durch das Fitting in Richtung auf das Ende des in der Körperhöhle befindlichen chirurgischen Instruments gepumpt, um diese aufzuwölben. Das Laparoskop wird durch einen speziellen Trokar positioniert, der eine hohle Leitung aufweist, die von seiner Spitze zu einem Fitting verläuft, das auf dem Abschnitt des Trokars angeordnet ist, der sich außerhalb der Körperhöhle befinden wird. Dieses Fitting ist mit einer Vakuumquelle verbunden. Das Rauchabzugssystem besitzt verschiedene äußere Elemente, die dazu benutzt werden, den Druck in der Körperhöhle zu überwachen, die CO₂-Strömung zum laserchirurgischen Instrument und den Zufluß vom laserchirurgischen Instrument zur Vakuumquelle zu steuern, sowie das Rauchabzugssystem zu aktivieren, wenn der schneidende Laserstrahl aktiviert wird. Optische Sensoren sind auf dem Fußschalter angeordnet, den der Chirurg normalerweise benutzt, um den schneidenden Laserstrahl zu aktivieren, wodurch mittels des Fußschalters zugleich die Aktivierung des Rauchabzugssystems gesteuert wird.

Bei Anwendung des Rauchabzugssystems nach der vorliegenden Erfindung wird eine Strömung von CO₂-Gas in Richtung des Laserstrahls automatisch jeweils dann erzeugt, wenn der Fußschalter betätigt wird, der den Laserschneidstrahl steuert. Gleichzeitig entfernt die Vakuumquelle den Rauch und den Wasserdampf aus der Körperhöhle durch den speziellen Trokar, wodurch die Qualität des Laserstrahls verbessert wird, nachdem Rauch und Wasserdampf den Laserstrahl absorbieren und ablenken können. Die Entfernung des Rauchs erfolgt demnach vollständig automatisch, und bedarf zum Betrieb des Rauchabzugssystems keines zusätzlichen Einschnitts und keines zusätzlichen Personals.

Anhand der nun folgenden Beschreibung eines in der Zeichnung dargestellten Ausführungsbeispiels der Erfindung wird diese näher erläutert.

Es zeigt:
- Fig. 1: eine schematisches Blockschaltbild eines erfindungsgemäßen automatischen Rauchabzugssystems und
- Fig. 2: eine Seitenansicht eines Laser-Lapraskops und eines speziellen Trokars, der als Teil des in Fig. 1 gezeigten automatischen Rauchabzugssystems benutzt wird, während des Gebrauchs in einer Körperhöhle bei einem laserchirurgischen Eingriff.

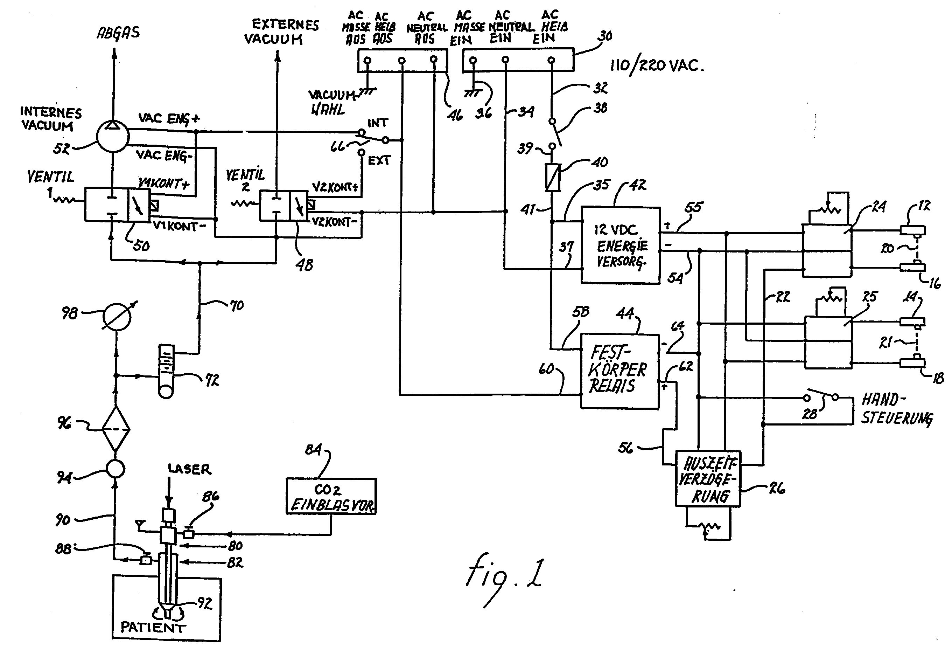

In Fig. 1 ist eine konventionelle Wechelstromquelle (AC) mit einer Eingangsklemmenleiste 30 verbunden, um die Energieversorgung des Rauchabzugssystems zu bilden. Dieser Wechselstromanschluß weist, wie gezeigt, einen Energieversorgungseingang 32, einen neutralen Eingang 34 und einen Erdungseingang 36 auf.

Der Wechselstrom-Energieversorgungseingang 32 ist mit einem EIN/AUS-Schalter 38 verbunden. Auf der anderen Seite 39 des EIN/AUS-Schalters 38 ist die zugeschaltete Energie mit einer Sicherung 40 verbunden. Auf der anderen Seite 41 der Sicherung 40 wird die abgesicherte Energie, wie gezeigt, mit dem positiven Eingang 35 einer 12 V-Gleichstrom-Versorgungseinheit 42 verbunden, sowie mit dem Eingang 48 eines Festkörperrelais 44.

Der neutrale Wechselstromeingang 34 ist, wie gezeigt, mit dem negativen Eingang 37 der 12 V-Gleichstrom-Versorgungseinheit 42 verbunden, sowie mit einer Ausgangsklemmenleiste 46, mit den negativen Steuereingängen von Magnetventilen 48 und 50 und mit einer Vakuumpumpe 52.

Der Wechselstrom-Erdungseingang 36 ist, wie gezeigt, mit dem Rahmen des Rauchabzugssystems und mit der Ausgangs-Klemmenleiste 46 verbunden.

Das Festkörperrelais 44 dient dazu, die abgesicherte Energie von dem mit dem Leitungsabschnitt 41 verbundenen Eingang 58 zum Ausgang 60 durchzuschalten, wenn ein logisches Hoch zwischen den Steueranschlüssen 62 und 64 vorhanden ist. Die auf den Ausgang 60 geschaltete Energie dient zur Versorgung der Vakuumpumpe 52 und der zur Steuerung des Abflusses von Rauch und CO₂ aus der Körperhöhle dienenden Magnetventile 48 und 50.

Ein Vakuumwählschalter 66 wird benutzt, um entweder eine externe Vakuumquelle oder das interne, durch die Vakuumpumpe 52 erzeugte Vakuum innerhalb des Rauchabzugssystems einzusetzen.

Wie aus dem Blockschaltbild zu entnehmen ist, wird der Ausgang 60 des Festkörperrelais 44 mit der Vakuumpumpe 52 und dem positiven Steueranschluß des Magnetventils 50 verbunden, wenn der Vakuumwählschalter 66 auf INTERN eingestellt ist. Wenn der Vakuumwählschalter 66 auf EXTERN eingestellt ist, wird der Ausgang 60 des Festkörperrelais 44 mit dem positiven Steueranschluß des Magnetventils 48 verbunden.

Der negative Ausgang 54 der 12 V-Gleichstromversorgungseinheit 42 ist, wie gezeigt, mit den negativen Energieeingängen eines optischen Sensors 24, eines optischen Sensors 25, des Festkörperrelais 44, eines Zeitglieds 26 und eines Handsteuerschalters 28 verbunden. Der positive Ausgang 55 der 12 V-Gleichstrom-Versorgungseinheit 42 ist, wie gezeigt, mit den positiven Energieeingängen des optischen Sensors 24, des optischen Sensors 25 und des Zeitglieds 26 verbunden.

Dieses System umfaßt optische Geber 12 und 14 mit zugeordneten optischen Empfängern 16 und 18. Der optische Geber 12 sendet einen optischen Strahl 20 zum optischen Empfänger 16. Gleichermaßen sendet der optische Geber 14 einen optischen Strahl 21 zum optischen Empfänger 18. Die optischen Geber 12 und 14 und die optischen Empfänger 16 und 18 sind alle derart dem Fußschalter zur Betätigung des Laserschneidstrahls zugeordnet gelagert, daß die Strahlen 20 und 21 unterbrochen werden, wenn sich der Fußschalter in einer vorgegebenen Position befindet. Das Auftreten oder das Fehlen des optischen Strahls 20 am optischen Empfänger 16 erzeugt am Signalausgang 22 des optischen Sensors 24 ein Steuersignal. Dieses Steuersignal dient zur Steuerung des Zeitglieds 26. Der optische Sensor 25 arbeitet gleichermaßen mit dem optischen Geber 16 und dem optischen Empfänger 18 zusammen.

Die Steuersignale der optischen Sensoren 24 und 25 am Signalausgang 22 werden benutzt, um das Zeitglied 26 zu triggern. Wenn der Fußschalter für den Laserschneidstrahl vom Chirurgen niedergedrückt wird, veranlaßt das Steuersignal im Signalausgang 22 das Auftreten eines logischen Hochs am Ausgang 56 des Zeitglieds 26. Dieses logische Hoch-Signal am positiven Steuereingang 62 des Festkörperrelais 44 veranlaßt dieses, die abgesicherte Energie vom Eingang 58 auf den Ausgang 60 durchzuschalten. Dies veranlaßt die Öffnung des durch den Vakuumwählschalter 66 ausgewählten Ventils. (Die beiden Magnetventile 48 und 50 sind in Ruhestellung geschlossen.) Falls der Vakuumwählschalter 66 sich in der Stellung INTERN befindet, wird die Öffnung des Ventils 50 von der Aktivierung der Vakuumpumpe 52 zur Erzeugung des internen Vakuums begleitet. Falls sich der Vakuumwählschalter 66 in der Stellung EXTERN befindet, wird das Ventil 48 geöffnet. In jedem Fall wird Energie auf die Ausgangsklemmenleiste 46 geschaltet, um eine externe Vakuumquelle zu aktivieren, falls dies erforderlich ist. Unabhängig davon, ob die Vakuumquelle in Bezug auf das Rauchabzugsystem intern oder extern ist, wird in der Vakuumleitung 70 ein Vakuum erzeugt.

Es ist zu beachten, daß der manuell betätigbare Steuerschalter 28 benutzt werden kann, um die optischen Sensoren 24 und 25 zu umgehen und das Steuersignal in Signalausgang 22 direkt zu erzeugen. Damit besteht die Möglichkeit, beim Rauchabzugssystem einen neuen Rauchabzugszyklus bei Anwendungen zu starten, die dies erfordern.

Das Zeitglied 26 erzeugt eine Zeitverzögerung, damit das Vakuum in der Leitung 70 nach der Freigabe des Fußschalters für den Laserschneidstrahl noch für eine Zeitspanne erhalten bleibt. Diese Verzögerung ermöglicht ein Absaugen nach der Beendigung des Schneidvorgangs, um sicherzustellen, daß der Rauch vollstandig abgezogen wird.

In Verbindung mit dem zugehörigen Trokar 82 ist ein Laparoskop 80 dargestellt. Der Laser für das Laparoskop 80 ist nicht gezeigt. Das Laparoskop 80 besitzt einen Stöpselhahn 86 für die Verbindung mit der CO₂-Einblasvorrichtung 84. Der Trokar 82 besitzt einen Stöpselhahn 88 zur Verbindung mit einer Vakuumleitung 90. Dieser Stöpselhahn 88 ist mit einer Anzahl von Löchern 92 am Boden des Trokars 82 verbunden, die den Absaugweg für das Rauchabzugssystem bilden.

Die Vakuumleitung 90 ist mit einem Wasserabscheider 94 und einem Filter 96 verbunden, die den größten Teil der Verunreinigungen aus dem Absaugstrom entfernen. Ein Druckmesser 98 wird eingesetzt, um den Druck in der Körperhöhle zu überwachen und ein einstellbarer Strömungsmesser 72 dient dazu, den Absaugstrom auf einem geeigneten Niveau zu halten.

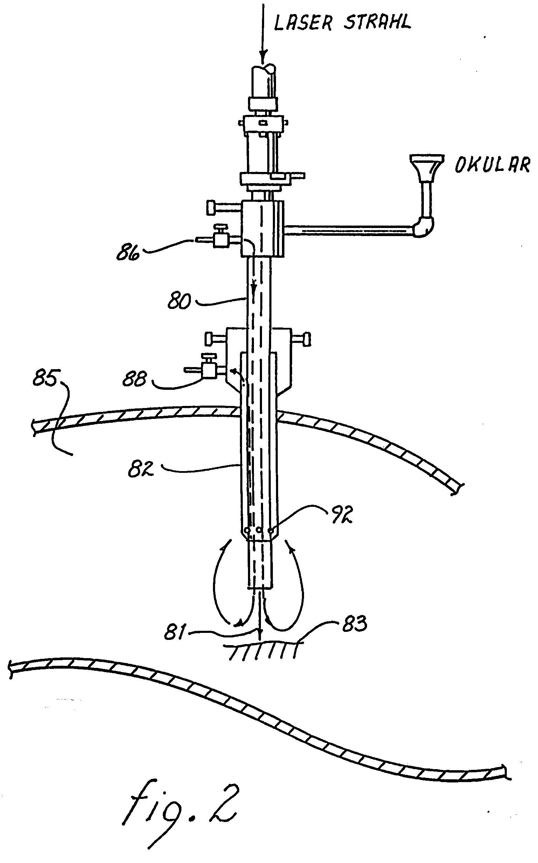

Die Fig. 2 zeigt das mit dem Trokar 82 ausgerüstete Laparoskop 80. Wie in Fig. 1 gezeigt, ist der Stöpselhahn 86 mit der CO₂-Einblasvorrichtung 84 und der Stöpselhahn 88 mit der Vakuumleitung 90 verbundene. Wie aus Fig. 2 ersichtlich kann bei aktiviertem Laserschneidstrahl 81 das Schneiden des Gewebes mit dem Laserstrahl Rauch und Wasserdampf erzeugen. Die Aktivierung des schneidenden Laserstrahls bewirkt automatisch die Aktivierung des Rauchabzugssystems. Wenn das Rauchabzugssystem aktiviert ist, tritt in der Leitung 90 am Stöpselhahn 88 ein Vakuum auf, das Rauch und CO₂-Gas durch die Löcher 92 aus der Körperhöhle des Patienten abzieht. Dieser Druckabfall ermöglicht es, wie gezeigt, dem CO₂, von der Einblasvorrichtung durch den Stöpselhahn 86 zum Ende des Laparoskops zu strömen. Zur gleichen Zeit werden aufgrund des Vakuums Rauch und Wasserdampf über die Löcher 92 abgezogen. Dieser Zustrom von frischem CO₂ zum Gewebe 82 und nach oben zu den Absauglöchern 92 verhilft dazu, die Linse des Laser-Laparoskops 80 von Verunreinigungen durch Rauch, Wasserdampf und Teilchen freizuhalten und dadurch das Arbeitsergebnis des Laser-Laparoskops zu verbessern.

Der Gebrauch des Rauchabzugssystems ist ausgehend von den normalen Aktivitäten des Chirurgen leicht durchschaubar, wenn das System einmal angeschlossen und gebrauchsfertig ist. Der Chirurg benutzt den Trokar, der mit Absauglöchern 92 versehen ist, die mit dem Stöpselhahn 88 verbunden sind. Dieser Stöpselhahn 88 ist wie gezeigt mit der Vakuumleitung 90 verbunden. Der Chirurg setzt dann das Laser-Laparoskop in den Trokar ein, worauf der Rauchabzug während der chirurgischen Tätigkeit automatisch jedesmal dann stattfindet, wenn der Laserstrahl aktiviert wird.

Falls im Operationssaal eine Vakuumquelle zur Verfügung steht, wird diese mit dem Ausgang des Magnetventils 48 verbunden. Der Vakuumwählschalter 66 des Rauchabzugssystems wird dann auf die EXTERN-Stellung eingestellt, weil eine außerhalb des Systems befindliche Vakuumquelle benutzt werden soll. Dadurch wird das Festkörperrelais 44 veranlaßt, das Ventil 48 zu aktivieren, sobald ein Rauchabzug erforderlich ist. Zur gleichen Zeit wird Wechselstromenergie auf die Ausgangsklemmenleiste 46 geschaltet, die dann mit dem Versorgungskabel der externen Vakuumquelle verbunden werden kann.

Falls im Operationssaal keine geeignete Vakuumquelle zur Verfügung steht, oder falls der Chirurg den Gebrauch der internen Vakuumquelle des Systems wünscht, wird der Vakuumwählschalter 66 in die INTERN-Stellung gebracht. Dadurch wird das Festkörperrelais 44 veranlaßt, zur Erzeugung des internen Vakuums die Vakuumpumpe 52 zu aktivieren, sowie gleichzeitig das Ventil 50 zu betätigen, wenn ein Rauchabzug benötigt wird.

Wenn der Chirurg den Fußschalter drückt, um den Laserstrahl zu aktivieren, stellt wenigstens einer der optischen Sensoren 24 und 25 die Fußbewegung fest und erzeugt im Ausgang 22 das Steuersignal. Dieses Steuersignal triggert das Zeitglied 26, welches den Steuereingang 62 des Festkörperrelais 44 hoch setzt. Dadurch wird das Festkörperrelais 44 veranlaßt, das durch den Vakuumwählschalter 66 ausgewählte Ventil mit Wechselstromenergie zu versorgen. Diese Wechselstromenergie am Eingang des Ventils veranlaßt die Öffnung des Ventils. Die Öffnung des entsprechenden Ventils hat zur Folge, daß durch das resultierende Vakuum Rauch und Gas aus der Körperhöhle über die Löcher im Trokar 82 und aus dem Stöpselhahn 88 zur Vakuumleitung 90 und durch den Wasserabscheider 94, den Filter 96, den einstellbaren Strömungsmesser 72 und die Vakuumleitung 70 strömen, sowie durch das durch den Vakuumwählschalter ausgewählte Ventil 48 oder 50 zu einer Auslaßöffnung. Wenn der Chirurg das Niederdrücken des Fußschalters zur Steuerung des Laserschneidstrahls beendet, verschwindet das Steuersignal im Sensorausgang 22, wodurch das Zeitglied 26 veranlaßt wird, die Zeitverzögerungssequenz zu beginnen, wodurch das Vakuum auf der Leitung 70 für eine gewisse Zeitspanne nach dem Verschwinden des Steuersignals im Sensorausgang 22 aufrechterhalten wird. Auf diese Weise wird nach dem Beenden des Schneidvorgangs der gesamte verbleibende Rauch entfernt, so daß nach jeder Betätigung des Laserschneidstrahls der Rauch jeweils vollständig entfernt wird.

Weil das Rauchabzugssystem nach der vorliegenden Erfindung nur dann aktiviert wird, wenn der Laserschneidstrahl aktiviert wird, ist das Rauchabzugssystem nur für einen Bruchteil der Zeit eingeschaltet, die der Patient in der chirurgischen Behandlung verbringt. Aus diesem Grund muß das CO₂-Gas nicht wiederaufbereitet werden, welches durch die Vakuumquelle aus dem Patienten entfernt wird. Bei dem Stand der Technik angehörenden Rauchabzugssystemen wird das System während der chirurgischen Behandlung kontinuierlich betrieben, wodurch eine Wiedergewinnung und Wiederaufbereitung des kontaminierten CO₂ erwünscht ist. Beim erfindungsgemäßen Rauchabzugssystem werden jedoch so geringe Mengen an CO₂ verwendet, daß das kontaminierte CO₂ gefiltert und außerhalb der Auslaßöffnung beseitigt werden kann. Dieses Verfahren stellt sicher, daß kein kontaminiertes Material auf den dem Verfahren unterworfenen Patienten übertragen werden kann, oder auf den nächsten Patienten, dessen Behandlung den Gebrauch des Rauchabzugssystems erfordert.

Obwohl die Erfindung anhand einer bevorzugten Ausführungsform beschrieben wurde, ist dies nicht beschränkend zu verstehen. Beispielsweise kann ein anderes chirurgisches Laserinstrument, wie etwa ein Laserinstrument für die Zahnbehandlung, verwendet werden.

## Patentansprüche

1. Chirurgische Laservorrichtung mit einem Rauchabzugssystem, die versehen ist mit
a) einer Gaszuführungsleitung in der chirurgischen Laservorrichtung (80),
b) einer mit der chirurgischen Laservorrichtung (80) gekuppelten Einrichtung (84, 86) zum Erzeugen einer Gasströmung in der Gaszuführungsleitung,
c) einer mit der chirurgischen Laservorrichtung (80) gekuppelten Gasausleitungseinrichtung (92, 82, 88, 90) zum Abzug von Gas aus dem Bereich der chirurgischen Laservorrichtung (80),
d) Vakuummitteln (52) zum Schaffen einer eine Gasströmung bewirkenden und auf die Gasausleitungseinrichtung (92, 82, 88, 90) einwirkenden Kraft, und
e) einer Gasleitungsanordnung (72, 94, 96) zum Verbinden der Vakuummittel (52) mit der Gasausleitungseinrichtung (92, 82, 88, 90),
**gekennzeichnet durch**
f) eine in der Gasleitungsanordnung (72, 94, 96) befindliche, elektrisch betätigbare Ventileinrichtung (48, 50) zum Steuern des Gasflusses in der Gasleitungsanordnung (72, 94, 96) und zur Schaltung der Vakuummittel (52), und
g) eine Steuereinrichtung (44, 60, 66) zum Betätigen der Ventileinrichtung (48, 50) in Abhängigkeit von der Betätigung der chirurgischen Laservorrichtung (80) und zur Schaffung eines automatischen Rauchabzugssystems.

2. Chirurgische Laservorrichtung nach Anspruch 1, **dadurch gekennzeichnet,** daß die chirurgische Laservorrichtung (80) ein Laser-Laparaskop umfaßt.

3. Chirurgische Laservorrichtung nach Anspruch 1 oder 2, **dadurch gekennzeichnet,** daß die Einrichtung (84, 86) zum Erzeugen einer Gasströmung in der Gasleitung der Laservorrichtung (80) eine Einblasvorrichtung (84) für Kohlendioxid aufweist.

4. Chirurgische Laservorrichtung nach Anspruch 1, **dadurch gekennzeichnet,** daß die Gasausleitungseinrichtung (92, 82, 88, 90) einen Trokar (82) umfaßt, der wenigstens eine Ausleitöffnung (92) an dem in eine Körperhöhle des Patienten einzusetzenden Ende aufweist und ein Lumen hat, das diese wenigstens eine Ausleitöffnung (92) mit einem Fitting (88) verbindet, das sich an dem außerhalb der Körperhöhle verbleibenden Ende des Trokars befindet und mit der Gasleitungsanordnung (72, 94, 96) verbunden ist.

5. Chirurgische Laservorrichtung nach Anspruch 1, **dadurch gekennzeichnet,** daß die Vakuummittel eine in das Rauchabzugssystem einbezogene Vakuumpumpe (82) umfassen.

6. Chirurgische Laservorrichtung nach Anspruch 1, **dadurch gekennzeichnet,** daß die Vakuummittel eine außerhalb des Rauchabzugssystems befindliche Vakuumquelle aufweisen.

7. Chirurgische Laservorrichtung nach Anspruch 1, **dadurch gekennzeichnet,** daß die Ventileinrichtung wenigstens ein durch die Steuereinrichtung (44, 60, 66) gesteuertes Magnetventil (48, 50) umfaßt.

8. Chirurgische Laservorrichtung nach Anspruch 1, **dadurch gekennzeichnet,** daß das Magnetventil (48, 50) in Ruhestellung geschlossen ist.

9. Chirurgische Laservorrichtung nach Anspruch 1, **dadurch gekennzeichnet**, daß die Gasleitungsanordnung (72, 94, 96) einen Wasserabscheider (94) umfaßt.

10. Chirurgische Laservorrichtung nach Anspruch 9, **dadurch gekennzeichnet**, daß die Gasleitungsanordnung (72, 94, 96) auch ein Filterelement (96) enthält.

11. Chirurgische Laservorrichtung nach Anspruch 9 oder 10, **dadurch gekennzeichnet**, daß die Gasleitungsanordnung (72, 94, 96) auch einen einstellbaren Strömungsmesser (72) enthält.

12. Chirurgische Laservorrichtung nach einem der Ansprüche 9 bis 11, **dadurch gekennzeichnet**, daß an die Gasleitungsanordnung (72, 94, 96) ein Druckmesser (98) angeschlossen ist.

13. Chirurgische Laservorrichtung nach Anspruch 1, **dadurch gekennzeichnet**, daß die Steuereinrichtung (44, 60, 66) versehen ist mit
a) einem Energieeingang (30),
b) wenigstens einer Steuerschaltereinrichtung (24, 25), die geeignet ist, die Betätigung der chirurgischen Laservorrichtung (80) festzustellen, und die einen zwei elektrische Zustände aufweisenden Ausgang besitzt, und
c) einem Leistungsschalter (44), der die Ventileinrichtung (48, 50) und die Vakuummittel (52) beim Auftreten eines von der Steuerschaltereinrichtung (24, 25) erzeugten Signals mit Energie versorgt.

14. Chirurgische Laservorrichtung nach Anspruch 13, **dadurch gekennzeichnet**, daß der Energieeingang (30) ein Dreileiter-Netzanschlußkabel aufweist, das mit einem Stecker versehen ist, der das Netzanschlußkabel mit einem konventionellen Energieausgang für Wechselstrom verbindet.

15. Chirurgische Laservorrichtung nach Anspruch 13, **gekennzeichnet** durch Mittel (42) zur Gleichspannungsregelung.

16. Chirurgische Laservorrichtung nach einem der Ansprüche 13 bis 15, **dadurch gekennzeichnet**, daß die Steuerschaltereinrichtung (24, 25) einen optischen Sensor mit einem optischen Geber (12, 14) und einem optischen Empfänger (16, 18) umfaßt, daß der Ausgang sich in einem der beiden elektrischen Zustände befindet, wenn der optische Strahl zwischen Geber (12, 14) und Empfänger (16, 18) nicht unterbrochen ist, und daß der Ausgang in den anderen Zustand überwechselt, wenn der optische Strahl unterbrochen wird.

17. Chirurgische Laservorrichtung nach Anspruch 16, **dadurch gekennzeichnet**, daß die Steuerschaltereinrichtung (24, 25) mechanisch derart mit einem die chirurgische Laservorrichtung (80) aktivierenden Schalter gekuppelt ist, daß die Betätigung dieses Schalters einen Wechsel des Zustands am Ausgang der Steuerschaltereinrichtung (24, 25) zur Folge hat.

18. Chirurgische Laservorrichtung nach Anspruch 17, **dadurch gekennzeichnet**, daß der die chirurgische Laservorrichtung (80) akitivierende Schalter ein Fußschalter ist.

19. Chirurgische Laservorrichtung nach einem der Ansprüche 16 bis 18, **dadurch gekennzeichnet**, daß das Signal am Ausgang der Steuerschaltereinrichtung (24, 25) geeignet ist, einen neuen Rauchabzugszyklus zu starten.

20. Chirurgische Laservorrichtung nach einem der Ansprüche 13 bis 19, **gekennzeichnet** durch ein Zeitgleid (26) zur Verzögerung des Aktivierungsendes des Leistungsschalters (44).

21. Chirurgische Laservorrichtung nach einem der vorhergehenden Ansprüche, **dadurch gekennzeichnet**, daß der Leistungsschalter (44) ein Festkörperrelais umfaßt.

22. Chirurgische Laservorrichtung nach Anspruch 13, **gekennzeichnet** durch einen vom Ausgang des Leistungsschalters (44) gebildeten Ausgang für die geschaltete Energie zur Versorung einer außerhalb des Rauchabzugssystems befindlichen Vakuumquelle.

23. Chirurgische Laservorrichtung nach einem der Ansprüche 13 bis 22, **gekennzeichnet** durch einen Vakuumwählschalter (66), der den elektrischen Steuereingang an einer der Ventileinrichtungen (48, 50) mit dem Ausgang des Leistungsschalters (44) verbindet, wenn er sich in einem ersten Schaltzustand befindet, und der den elektrischen Steuereingang der zweiten Ventileinrichtung (48, 50) mit dem Ausgang des Leistungsschalters (44) verbindet, wenn er sich in einem zweiten Schaltzustand befindet.

## Claims

1. Surgical laser device with a smoke extraction system, which device is provided with
a) a gas feed duct in the surgical laser device (80),
b) an equipment (84, 86), which is coupled with the surgical laser device (80), for the production of a gas flow in the gas feed duct,
c) a gas outlet duct equipment (92, 82, 88, 90), which is coupled with the surgical laser device (80), for the extraction of gas from the region of the surgical laser device (80),
d) vacuum means (52) for the creating of a force acting on the gas outlet duct equipment (92, 82, 88, 90) and causing a gas flow and
e) a gas duct arrangement (72, 94, 96) for the connecting of the vacuum means (52) with the gas outlet duct equipment (92, 82, 88, 90),
characterised by
f) an electrically actuatable valve equipment (48, 50), which is disposed in the gas duct arrangement (72, 94, 96), for the controlling of the gas flow in the gas duct arrangement (72, 94, 96) and for the switching of the vacuum means (52) and
g) a control equipment (44, 60, 66) for the actuation of the valve equipment (48, 50) in dependence on the actuation of the surgical laser device (80) and for the creating of an automatic smoke extraction system.

2. Surgical laser device according to claim 1, characterised thereby, that the surgical laser device (80) comprises a laser laparoscope.

3. Surgical laser device according to claim 1 or 2, characterised thereby, that the equipment (84, 86) for the producing of a gas flow in the gas duct of the laser device (80) displays an injection device (84) for carbon dioxide.

4. Surgical laser device according to claim 1, characterised thereby, that the gas outlet duct equipment (92, 82, 88, 90) comprises a trocar (82), which displays at least one outlet opening (92) at the end to be inserted into a body cavity of the patient and has a lumen which connects this at least one outlet opening (92) with a fitting (88), which is disposed at that end of the trocar, which remains outside the body cavity, and is connected with the gas duct arrangement (72, 94, 96).

5. Surgical laser device according to claim 1, characterised thereby, that the vacuum means comprise a vacuum pump included in the smoke extraction system.

6. Surgical laser device according to claim 1, characterised thereby, that the vacuum means display a vacuum source disposed externally of the smoke extraction system.

7. Surgical laser device according to claim 1, characterised thereby, that the valve equipment comprises at least one magnetic valve (48, 50) controlled by the control equipment (44, 60, 66).

8. Surgical laser device according to claim 1, characterised thereby, that the magnetic valve (48, 50) is closed in the rest setting.

9. Surgical laser device according to claim 1, characterised thereby, that the gas duct arrangement (72, 94, 96) comprises a water separator (94).

10. Surgical laser device according to claim 9, characterised thereby, that the gas duct arrangement (72, 94, 96) also contains a filter element (96).

11. Surgical laser device according to claim 9 or 10, characterised thereby, that the gas duct arrangement (72, 94, 96) also contains an adjustable flow meter (72).

12. Surgical laser device according to one of the claims 9 to 11, characterised thereby, that a pressure meter (98) is connected to the gas duct arrangement (72, 94, 96).

13. Surgical laser device according to claim 1, characterised thereby, that the control equipment (44, 60, 66) is provided with
a) an energy input (30),
b) at least one control switch equipment (24, 25), which is suitable for ascertaining the actuation of the surgical laser device (80) and which has an output displaying two electrical states and
c) a power switch (44), which supplies the valve equipment (48, 50) and the vacuum means (52) with energy on the occurrence of a signal produced by the control switch arrangement (24, 25).

14. Surgical laser device according to claim 13, characterised thereby, that the energy input (30) displays a three-core mains connection cable which is provided with a plug which connects the mains connection cable with a conventional energy output for alternating current.

15. Surgical laser device according to claim 13, characterised by means (42) for direct voltage regulation.

16. Surgical laser device according to one of the claims 13 to 15, characterised thereby, that the control switch arrangement (24, 25) comprises an optical sensor with an optical transmitter (12, 14) and an optical receiver (16, 18), that the output is in one of both the electrical states when the optical beam between transmitter (12, 14) and receiver (16, 18) is not interrupted and that the output changes over into the other state when the optical beam is interrupted.

17. Surgical laser device according to claim 16, characterised thereby, that the control switch arrangement (24, 25) is coupled mechanically in such a manner with a switch activating the surgical laser device (80) that the actuation of this switch has the consequence of a change in the state at the output of the control switch arrangement (24, 25).

18. Surgical laser device according to claim 17, characterised thereby, that the switch activating the surgical laser device (80) is a pedal switch.

19. Surgical laser device according to one of the claims 16 to 18, characterised thereby, that the signal at the output of the control switch arrangement (24, 25) is suitable for starting a new smoke extraction cycle.

20. Surgical laser device according to one of the claims 13 to 19, characterised by a timing member (26) for delaying the end of activation of the power switch (44).

21. Surgical laser device according to one of the preceding claims, characterised thereby, that the power switch (44) comprises a solid state relay.

22. Surgical laser device according to claim 13, characterised by an output, which is formed by the output of the power switch (44), for the switched energy for the supply of a vacuum source disposed externally of the smoke extraction system.

23. Surgical laser device according to claims 13 to 22, characterised by a vacuum selector switch (66), which connects the electrical control input at one of the valve equipments (48, 50) with the output of the power switch (44) when it is in a first switching state and which connects the electrical control input of the second valve equipment (48, 50) with the output of the power switch (44) when it is in a second switching state.

## Revendications

1. Dispositif laser chirurgical comportant un système de cheminée, qui est muni :
a) d'une conduite d'amenée de gaz dans le dispositif (80) de laser chirurgical
b) d'un dispositif (84,86) accouplé au dispositif (80) laser chirurgical et destiné à la production d'un courant de gaz dans la conduite d'amenée du gaz,
c) d'un dispositif (92,82,88,90) d'évacuation de gaz accouplé au dispositif (80) laser chirurgical et destiné à l'évacuation de gaz de la zone du dispositif (80) laser chirurgical,
d) des moyens (52) de dépression destinés à créer une force provoquant un courant de gaz et agissant sur le dispositif (92,82,88,90) d'évacuation de gaz, et,
e) d'un dispositif (72,94,96) de conduite de gaz pour faire communiquer les moyens (52) de dépression avec le dispositif (92,82,88,90) d'évacuation de gaz,
**caractérisé par**
f) un dispositif (48,50) de vanne pouvant être actionné électriquement, se trouvant dans le dispositif (72,94,96) de conduite de gaz, destiné à la commande du flux de gaz dans le dispositif (72, 94,96) de conduite de gaz et destiné à brancher les moyens (52) de dépression, et
g) un dispositif (44,60,66) de commande destiné à actionner le dispositif (48,50) de vanne en fonction de l'actionnement du dispositif (80) laser chirurgical et destiné à créer un système automatique de cheminée.

2. Dispositif laser chirurgical suivant la revendication 1, **caractérisé en ce que** le dispositif laser chirurgical (80) comprend un laparoscope laser.

3. Dispositif laser chirurgical suivant la revendication 1 ou 2, **caractérisé en ce que** le dispositif (84,86) de production d'un courant de gaz dans la conduite de gaz du dispositif (80) laser comporte un dispositif (84) d'insufflation de dioxyde de carbone.

4. Dispositif laser chirurgical suivant la revendication 1, **caractérisé en ce que** le dispositif (92,82,88,90) d'évacuation de gaz comprend un trocart (82), qui comporte au moins une ouverture (92) d'évacuation à l'extrémité à introduire dans une cavité du corps du patient et qui a un calibre qui relie au moins une ouverture (92) d'évacuation à un raccord (88) à collerette, qui se trouve à l'extrémité du trocart restant à l'extérieur de la cavité du corps et qui communique avec le dispositif (72,94,96) de conduite de gaz.

5. Dispositif laser chirurgical suivant la revendication 1, **caractérisé en ce que** le moyen de dépression comprend une pompe (82) à vide incorporée au système de cheminée.

6. Dispositif laser chirurgical suivant la revendication 1, **caractérisé en ce que** les moyens de dépression comportent une source sous vide se trouvant à l'extérieur du système de cheminée.

7. Dispositif laser chirurgical suivant la revendication 1, **caractérisé en ce que** le dispositif de vanne comprend au moins une vanne magnétique (48,50) commandée par le dispositif (44,60,66) de commande.

8. Dispositif laser chirurgical suivant la revendication 1, **caractérisé en ce que** la vanne magnétique (48,50) est fermée dans la position de repos.

9. Dispositif laser chirurgical suivant la revendication 1, **caractérisé en ce que** le dispositif (72,94,96) de conduite de gaz comprend un séparateur (94) d'eau.

10. Dispositif laser chirurgical suivant la revendication 9, **caractérisé en ce que** le dispositif (72,94,96) de conduite de gaz comprend également un élément (96) de filtration.

11. Dispositif laser chirurgical suivant la revendication 9 ou 10, **caractérisé en ce que** le dispositif (72,94,96) de conduite de gaz comprend également un rhéomètre (72) réglable.

12. Dispositif laser chirurgical suivant l'une des revendications 9 à 11, **caractérisé en ce qu'**un manomètre (98) communique avec le dispositif (72,94,96) de conduite de gaz.

13. Dispositif laser chirurgical suivant la revendication 1, **caractérisé en ce que** le dispositif (44,60,66) de commande est muni de
a) une entrée (30) d'énergie,
b) au moins un dispositif de commutation de commande (24,25), qui est approprié pour constater l'actionnement du dispositif laser (80) chirurgical et qui comprend une sortie ayant deux états électriques, et
c) un interrupteur (44) de puissance, qui alimente en énergie le dispositif (48,50) de vanne et les moyens (52) de dépression lorsqu'il apparaît un signal produit par le dispositif (24,25) d'interruption de commande.

14. Dispositif laser chirurgical suivant la revendication 13, **caractérisé en ce que** l'entrée (30) d'énergie comporte un câble de raccordement au réseau à 3 fils, qui est muni d'une prise reliant le câble de raccordement au réseau à une sortie d'énergie classique de courant alternatif.

15. Dispositif laser chirurgical suivant la revendication 13, **caractérisé par** des moyens (42) de régulation de la tension continue.

16. Dispositif laser chirurgical suivant l'une des revendications 13 à 15, **caractérisé en ce que** le dispositif (24,25) d'interruption de commande comprend un capteur optique ayant un émetteur optique (12,14) et un récepteur optique (16,18), la sortie se trouve dans l'un des deux états électriques, lorsque le faisceau optique entre l'émetteur (12,14) et le récepteur (16,18) n'est pas interrompu et la sortie passe à l'autre état, lorsque le faisceau optique est interrompu.

17. Dispositif laser chirurgical suivant la revendication 16, **caractérisé en ce que** le dispositif (24,25) d'interruption de commande est accouplé mécaniquement à un interrupteur activant le dispositif (80) laser chirurgical, de telle sorte que l'actionnement de cet interrupteur a pour conséquence un changement de l'état à la sortie du dispositif (24,25) d'interruption de commande.

18. Dispositif laser chirurgical suivant la revendication 17, **caractérisé en ce que** l'interrupteur activant le dispositif laser chirurgical (80) est un interrupteur à commande au pied.

19. Dispositif laser chirurgical suivant l'une des revendications 16 à 18, **caractérisé en ce que** le signal à la sortie du dispositif (24,25) d'interruption de commande est approprié pour commencer un nouveau cycle d'évacuation de fumée.

20. Dispositif laser chirurgical suivant l'une des revendications 13 à 19, **caractérisé par** un élément temporel (26) pour le retardement de la fin de l'activation de l'interrupteur (44) de puissance.

21. Dispositif laser chirurgical suivant l'une des revendications précédentes, **caractérisé en ce que** l'interrupteur (44) de puissance comprend un relais à semi-conducteur.

22. Dispositif laser chirurgical suivant la revendication 13, **caractérisé par** une sortie, formée par la sortie de l'interrupteur (44) de puissance, pour l'énergie transmise pour alimenter une source sous vide se trouvant à l'extérieur du système de cheminée.

23. Dispositif laser chirurgical suivant l'une des revendications 13 à 22, **caractérisé par** un commutateur (66) de sélection de vide, qui relie l'entrée de commande électrique à l'un des dispositifs (48,50) de vanne à la sortie de l'interrupteur (44) de puissance, lorsqu'il se trouve dans un premier état de commutation, et qui relie l'entrée de commande électrique du second dispositif (48,50) de vanne à la sortie de l'interrupteur (44) de puissance, lorsqu'il se trouve dans un second état de commutation.
